(19) Europäisches Patentamt
European Patent Office
Office européen des brevets

(11) Numéro de publication : **0 039 294**
**B1**

(12) # FASCICULE DE BREVET EUROPÉEN

(45) Date de publication du fascicule du brevet : **29.02.84**

(51) Int. Cl.$^3$ : **C 07 C 93/187**, A 61 K 31/215

(21) Numéro de dépôt : **81400671.4**

(22) Date de dépôt : **28.04.81**

(54) **Bis(phénoxyacétates substitués) de N-alkyl-dialcanolamines, procédé d'obtention, et compositions pharmaceutiques les contenant.**

(30) Priorité : **30.04.80 FR 8009886**

(43) Date de publication de la demande : **04.11.81 Bulletin 81/44**

(45) Mention de la délivrance du brevet : **29.02.84 Bulletin 84/09**

(84) Etats contractants désignés : **AT BE CH DE FR GB IT LI LU NL SE**

(56) Documents cités :
**BE-A- 674 556**
**FR-A- 2 085 634**
**US-A- 2 771 477**
**CHEMICAL ABSTRACTS, vol. 70, no. 9, 03-03-1969, page 360, abrégé 37836y Columbus, Ohio, US**

(73) Titulaire : **ANVAR Agence Nationale de Valorisation de la Recherche**
**43, rue de Caumartin**
**F-75436 Paris Cedex 09 (FR)**

(72) Inventeur : **Godfroid, Jean-Jacques**
**182 rue des Pyrénées**
**F-75020 Paris (FR)**
Inventeur : **Thuillier, Jean**
**6, 12 Rue Raffet**
**F-75016 Paris (FR)**

(74) Mandataire : **Phélip, Bruno et al**
**c/o Cabinet Harlé & Phélip 21, rue de la Rochefoucauld**
**F-75009 Paris (FR)**

**EP 0 039 294 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Bis(phénoxyacétates substitués) de N-alkyl-dialcanolamines, procédé d'obtention et compositions pharmaceutiques les contenant

La présente invention concerne des bis(phénoxyacétates substitués) de N-alkyl-dialcanolamines, un procédé pour leur obtention et leurs applications à titre de médicaments.

On connaît déjà des dérivés de phénoxyacétates substitués, qui présentent des propriétés pharmacologiques, et en particulier le 4-chloro-phénoxyacétate de diméthylaminoéthyle, qui est couramment dénommé « Méclofénoxate ». Ce composé, qui est utilisé comme régulateur métabolique central, a été décrit dans le brevet FR 1.359.614 et le brevet FR « spécial de médicament » 398 M.

On connaît également d'autres esters d'acides aryloxyalcanoïques. A cet effet, on peut citer le brevet US 2.771.477, relatif à des compositions herbicides faiblement volatiles contenant à titre d'ingrédient actif des esters d'acides aryloxyalcane-carboxyliques et d'alcanolamines contenant au moins un groupe hydroxy libre.

Le brevet BE 674.556 concerne les esters de polyols et d'acides alpha-aryloxy-alcanoïques appropriés pour le traitement de l'hypercholestérinémie.

Le brevet FR 70.13.346, publié sous le n° 2.085.634, concerne des esters polyfonctionnels de l'acide 2-(p-chloro-phénoxy)2-méthyl-propionique et leur utilisation comme médicaments faisant preuve d'une activité hypolipémiante et hypocholestérolémiante.

On notera que les produits ayant des propriétés hypocholestérolémiantes décrits dans ces documents ont une structure aromatique très lipophile. Ces produits sont tellement lipophiles qu'ils ne peuvent pas franchir la barrière hémato-encéphalée.

On a maintenant trouvé une nouvelle série de composés chimiques, qui sont des stimulants de l'activité nerveuse centrale, capables de s'opposer aux troubles de la cellule nerveuse et de son métabolisme. Ces composés ont également un effet anti-agrégant plaquettaire et un effet diurétique.

Les composés selon la présente invention sont des bis(phénoxyacétates substitués) de N-alkyl-dialcanolamines, qui répondent à la formule générale ci-après

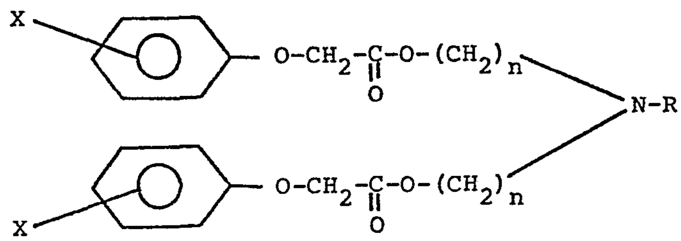

(I)

dans laquelle

— X est le chlore, le fluor ou le trifluorométhyle,

— n est compris entre 1 et 3 et est de préférence égal à 2,

— R est un groupe alkyle inférieur, tel que le groupe méthyle ou éthyle.

L'invention concerne également les sels pharmaceutiquement acceptables des composés de formule I.

L'invention a également pour objet un procédé pour l'obtention des composés de formule I. Ce procédé consiste :

1. à faire réagir, l'acide phénoxyacétique de formule

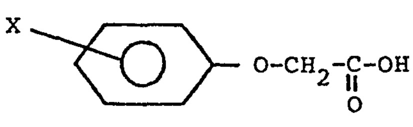

(II)

ou un de ses dérivés, avec une N-alkyl-dialcanolamine de formule

$$R—N—[(CH_2)_nOH]_2 \qquad (III)$$

dans un rapport de l'acide à l'alcool sensiblement de 2 : 1, X, R et n étant tels que définis ci-dessus,

2. à transformer éventuellement le composé obtenu en un sel pharmaceutiquement acceptable.

A titre de dérivés de l'acide on peut utiliser l'ester, le chlorure d'acide ou l'anhydride.

On opère avantageusement avec le chlorure de l'acide phénoxyacétique de formule II dans un solvant organique aliphatique ou aromatique approprié, par exemple le benzène, à reflux du solvant. Le produit résultant de la réaction de ce chlorure d'acide avec l'amine de formule III est donc un chlorhydrate qui peut être transformé par des moyens classiques en l'amine libre correspondante, par exemple par réaction avec du carbonate de sodium. L'amine libre ainsi obtenue est ensuite éventuellement transformée en un sel pharmaceutiquement acceptable par réaction avec un acide dans des

conditions classiques. A titre d'acides appropriés, on citera l'acide maléique, l'acide fumarique, l'acide oxalique, l'acide succinique, l'acide citrique, l'acide méthane-sulfonique et similaires.

Le composé particulièrement préféré selon l'invention est le bis-(4-chloro-phénoxyacétate) de N-méthyl-diéthanolamine.

Les composés selon l'invention ont une action remarquable sur la physiologie cérébrale et en particulier sur les régions diencéphalique et hypothalamique.

Des essais réalisés avec le bis-(4-chloro-phénoxyacétate) de N-méthyl-diéthanolamine ont un effet montré qu'il s'oppose aux œdèmes cérébraux et aux troubles provoqués par l'hypocapine. Il améliore le métabolisme cérébral par action sur les cellules nerveuses. Son action peut être vérifiée par des tests faisant intervenir la physiologie de l'hypothalamus, la stimulation des chromatophores et des modifications des conduites de faim et de soif.

Dans des protocoles expérimentaux, le bis-(4-chlorophénoxyacétate) de N-méthyl-diéthanolamine s'est révélé de 2 à 10 fois plus actif que le « Méclofénoxate » qui, lui-même, s'est révélé inactif dans certains essais où le composé préféré selon l'invention était très actif.

Les composés de formule (I) conviennent à la prévention et au traitement des troubles cérébraux dans les intoxications et les comas, les accidents vasculaires et les dérèglements des fonctions diencéphalohypothalamo-hypophysaires.

Enfin, l'invention concerne les compositions pharmaceutiques contenant à titre d'ingrédient actif un composé de formule I en combinaison avec un véhicule pharmaceutique acceptable.

Le véhicule pharmaceutiquement acceptable est choisi parmi les véhicules classiques couramment utilisés dans le domaine des médicaments. Il doit être inerte vis-à-vis des composés de l'invention.

Les compositions pharmaceutiques selon l'invention peuvent se présenter sous la forme de compositions administrables par voie orale ou parentérale.

Les compositions pharmaceutiques particulièrement préférées contiennent de 0,05 g à 1,50 g de bis-(4-chloro-phénoxyacétate) de N-méthyl-diéthanolamine.

L'invention va être maintenant illustrée sans être aucunement limitée par les exemples ci-après :

Exemple 1

Préparation du bis-(4-chloro-phénoxy-acétate) de N-méthyl diéthanolamine.

On a versé, goutte-à-goutte, 128 g de chlorure d'acide parachlorophénoxyacétique dans un mélange de 30 g de N-méthyl-diéthanolamine, fraîchement distillée, dans 100 $cm^3$ de benzène. Le mélange a été porté ensuite à reflux du benzène, avec une forte agitation pendant deux heures. Après retour à température ordinaire, les cristaux du chlorhydrate du composé recherché ont été essorés, lavés successivement à l'éther anhydre et à l'acétone tiède.

Après séchage à environ 100 °C sous vide, on a obtenu 96 g du chlorhydrate du produit recherché ayant un point de fusion F = 145-146 °C (méthode au capillaire). Rendement : 84 %. Ce produit peut être recristallisé dans l'éthanol ou l'isopropanol. Il existe sous une seconde forme cristalline dont le point de fusion (Köpfler) est de 116-120 °C.

Le sel obtenu était une poudre blanche insoluble dans l'éther et l'acétone et soluble dans l'eau tiède.

Analyse : $C_{21}H_{24}Cl_3NO_6$

Calculé : C 51,32 H 4,88 N 2,85 Cl 21,38

Trouvé : C 51,61 H 4,85 N 2,85 Cl 21,27

Infra-rouge : bande ester à 1 730 $cm^{-1}$.

RMN, δ (ppm) : 2,86, singulet $CH_3—\overset{+}{N}$, et 4,93, $O—CH_2—CO—$

Spectrographie de masse : ion moléculaire $M^+$ à 454 (poids moléculaire H—HCl).

Préparation de sels de PM 198 et obtention de la base du PM 198

Le chlorhydrate obtenu ci-dessus a été repris par la soude ou le carbonate de sodium aqueux. On a extrait la phase aqueuse à l'éther, on a lavé la solution organique à l'eau, puis on a séché cette solution. On a évaporé ensuite pour obtenir le PM 198 base.

Le PM 198 base a été repris dans un minimum d'acétone et on a versé une quantité stœchiométrique d'acide méthane sulfonique. Le méthane sulfonate de PM 198 a alors précipité. Il a été essoré et recristallisé dans le méthanol ou l'éthanol.

Après deux recristallisations dans le méthanol, le méthane sulfonate de PM 198 avait un point de fusion de 133 °C (Köpfler).

L'analyse de ce méthane sulfonate était la suivante :

Analyse :

Calculé : C 49,43 H 4,15 N 2,50 Cl 6,34

Trouvé : C 49,53 H 4,06 N 2,54 Cl 6,29

On a opéré selon le même mode opératoire que ci-dessus pour préparer le maléate et l'oxalate de PM 198 qui présentaient les points de fusion ci-après après recristallisation dans l'éthanol.

Maléate : 111 °C (Köpfler).

Oxalate : 137 °C (Köpfler).

On notera que le PM 198 base peut être obtenu directement en ajoutant, dans le mélange chlorure d'acide parachlorophénoxyacétique (2 moles)-N-méthyldiéthanolamine (1 mole)-benzène, deux moles d'une base capteur de protons, par exemple la triéthylamine ou la pyridine. Le chlorhydrate de ces bases ajoutées précipite alors dans le milieu réactionnel.

Exemple 2

On a opéré selon le mode opératoire de l'exemple 1 pour préparer le bis-(3-trifluorométhylphénoxyacétate) de N-méthyl-diéthanolamine de formule

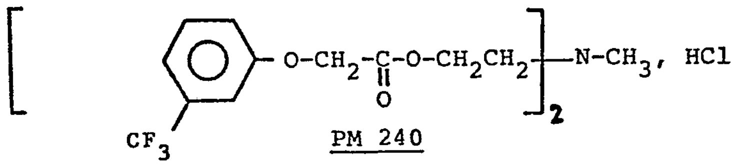

PM 240

F = 96° (Köpfler), solvant de recristallisation : alcool isopropylique/ligroïne.

Analyse :

Calculé : C 49,43 H 4,15 N 2,50 Cl 6,34

Trouvé : C 49,53 H 4,06 N 2,54 Cl 6,29

Exemple 3

Essais pharmacologiques

Le bis-(4-chloro-phénoxyacétate) de N-méthyldiéthanolamine obtenu selon l'exemple 1 a été soumis à différents essais pharmacologiques et toxicologiques.

Du fait de son analogie chimique avec le 4-chloro-phénoxyacétate de diméthylaminoéthyle (Méclofenoxate, de formule)

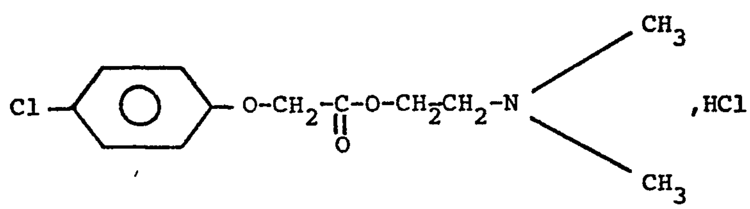

des essais comparatifs ont été effectués avec ces deux composés.

Le chlorhydrate bis-(4-chloro-phénoxyacétate) de N-méthyl-diéthanolamine selon l'invention sera dénommé dans la suite de la présente description, à des fins de simplicité, par l'abréviation « PM 198 ».

Essai A

Toxicité du bis-(4-chloro-phénoxyacétate) de N-méthyl-diéthanolamine et du Méclofénoxate.

Les toxicités ont été étudiées chez la souris par voies orale et intra-veineuse.

Les calculs de toxicité ont été effectués selon la méthode de Litchfield et Wilcoxon (J. Pharm. and Exp. Thérap. 1949-95, p. 99, 115)

| Produits | DL 50 P. O. mg/kg | DL 50 I. V. mg/kg |
|---|---|---|
| Méclofénoxate | 1 750 | 400 |
| PM 198 | 1 750 | 435 |

P. O. : voie orale.

I. V. : voie intraveineuse.

Les deux composés ont des toxicités sensiblement identiques.

Essai B

Action sur le système nerveux central

I. Action sur le cerveau

1. Test au triéthylétain

Les composés alkylés de l'étain sont toxiques et provoquent un œdème sélectif du système nerveux central et en particulier du cerveau [Katzman et al., Arch. Neurol. 9, 178, 1963]. Ces composés alkylés de l'étain augmentent la teneur en eau du tissu cérébral. De même, le contenu cérébral en sodium augmente alors que le potassium diminue.

On a trouvé qu'à la dose de 50 mg par kg, le PM 198 diminue l'œdème cérébral chez le rat traité au triéthylétain. Par contre, il faut 100 mg/kg de « Méclofénoxate » pour obtenir le même résultat.

De plus, on a noté que le PM 198 est beaucoup plus actif sur le syndrome neurologique que le Méclofénoxate, les rats gardant une attitude proche de la normale.

2. Test des électrochocs répétitifs chez le rat

Des électrochocs successivement pratiqués chez le rat et répétés trois fois à des intervalles de 20 minutes entraînent des troubles métaboliques cérébraux caractérisés par une perte de réflexes d'orientation et une mauvaise réponse aux stimulus douloureux.

Un traitement préventif utilisant l'administration 60 minutes avant le premier électrochoc de PM 198 a permis d'obtenir une protection très efficace vis-à-vis de ce test.

En effet, on a trouvé que l'administration intragastrique de 100 mg/kg de PM 198 améliorait significativement la réponse d'orientation dès le premier électrochoc.

Lors des deuxième et troisième électrochocs, les indices d'orientation sont encore supérieurs et de façon significative à ceux des animaux témoins (P = 0,01).

Le Méclofénoxate, même à la dose de 200 mg/kg reste absolument inactif pour ce test.

3. Action du PM 198 sur le syndrome de souffrance cérébrale du chien provoqué par l'hyperventilation entraînant l'hypocapnie.

L'hypocapnie provoquée chez le chien entraîne une souffrance cérébrale qui est mesurée :

— par l'étude de la disponibilité de l'oxygène,
— par la différence artério-veineuse en oxygène,
— par la consommation en oxygène/minute.

On étudie aussi la pression de perfusion et la résistance vasculaire cérébrale suivant le rapport :

R.V.C. = Pression systolique/Débit 1/minute

Le PM 198, à la dose de 50 mg/kg I.V. perfusé chez le chien en 20 minutes, s'est opposé, pendant une heure, à toute variation du débit veineux cérébral.

La pression veineuse en oxygène est restée normale et la consommation en oxygène a augmenté. Il n'y a pas eu de décompensation.

Le Méclofénoxate, à la dose de 50 mg/kg I.V. ne s'est opposé que pendant 20 minutes à la baisse du débit sanguin cérébral, mais n'a pas pu maintenir la pression en oxygène du cortex cérébral. Il y a eu décompensation.

II. Action sur le diencéphale

1. Action sur les chromatophores du poisson.

La physiologie des chromatophores de poissons sont sous la dépendance de sécrétions hypothalamiques de la partie inférieure du diencéphale. Les drogues psychotropes et à orientation cérébrale peuvent modifier la physiologie de ces régions [J. Thuillier et al., C.R. Soc. Biol. 1961, 155, 10 p., 1924-1928]. On sait que ce test démontre l'action des produits sur le diencéphale qui règle la dilatation et le noircissement des chromatophores de poissons.

On a utilisé le Phoxinus Phoxinus Linné immergé dans un aquarium renfermant une concentration choisie de PM 198. On a mesuré le temps d'apparition de noircissement du poisson. On a réalisé le même test avec le Méclofénoxate. Les résultats obtenus figurent dans le tableau I ci-après.

Tableau I

| Produit | Concentration dans l'eau | Temps minimum de noircissement secondes |
|---|---|---|
| PM 198 | 0,01 pour 1 000 | 90 |
| | 0,05 pour 1 000 | 45 |
| | 0,10 pour 1 000 | 25 |
| Méclofénoxate | 0,05 pour 1 000 | rien |
| | 0,10 pour 1 000 | rien |
| | 0,25 pour 1 000 | 90 |

Déjà à la concentration de 0,01 pour 1 000, le PM 198 a entraîné chez le Phoxinus Phoxinus Linné un noircissement, alors qu'il a fallu une dose 25 fois plus forte pour obtenir le même résultat avec le Méclofénoxate.

2. Action sur les conduites de faim.

La régulation de la faim et de la soif sont sous la dépendance de stimulations sécrétrices de l'hypothalamus. Ces centres régulateurs peuvent être perturbés soit de façon acquise chez des animaux génétiquement obèses, soit à la suite de destruction des centres considérés par des poisons sélectifs tels que l'aurothioglucose.

On a étudié l'action du PM 198 sur 2 types de souris hyperphagiques obèses, les unes génétiquement obèses, les autres intoxiquées par l'aurothioglucose.

2-1 Action du PM 198 sur des souris hyperphagiques par aurothioglucose

L'administration de 250 mg/kg d'aurothioglucose à la souris a provoqué une boulimie avec dilatation de l'estomac et une augmentation régulière du poids de l'animal par rapport à des témoins. Les résultats obtenus figurent dans le tableau II ci-après.

Tableau II

Action du PM 198 sur le poids de l'estomac

| Produits | Dose mg/kg P. O. | Diminution du poids des estomacs Statistiques Test t. | |
|---|---|---|---|
| | | Par rapport aux témoins à l'aurothioglucose | Degré de signification |
| PM 198 | 100 | t = 2,384 7 | P < 0,05 |
| | 150 | t = 2,312 6 | P < 0,05 |
| Méclofénoxate | 250 | t = 2,249 5 | P < 0,05 |

Les résultats du tableau II montrent qu'à la dose de 100 mg/kg le PM 198 a entraîné une diminution significative du poids des estomacs des souris traitées à l'aurothioglucose.

Pour obtenir ce même résultat, il a fallu utiliser une dose de 250 mg/kg de Méclofénoxate.

On a mesuré également l'augmentation respective des poids des souris traitées et non traitées par le PM 198 et le Méclofénoxate. Les résultats obtenus figurent dans le tableau III ci-après :

(voir Tableau III, page 7)

Tableau III

Action du PM 198 sur le développement de l'obésité induite par l'aurothioglucose

| Produit 100 mg/kg | Poids initial | Poids après 15 j. | Prise pondérale | Prise pondérale en % |
|---|---|---|---|---|
| Témoin absolu | 199 | 24,89 | 5,89 | 30 % |
| Témoin aurothioglucose | 219 | 32,85 | 11,85 | 57 % |
| PM 198 | 20,38 | 27,75 | 7,37 | 36 % |
| Méclofénoxate | 20,00 | 30,2 | 10,8 | 54 % |

A la dose de 100 mg/kg, le PM 198 a diminué la prise pondérale et s'est opposé à l'obésité induite par l'aurothioglucose. A cette même dose, le Méclofénoxate n'a pas été efficace.

2-2 Action du PM 198 sur l'obésité génétique chez la souris

Il existe des souches de souris obèses (Elevage du Centre National de la Recherche Scientifique, Orléans).

Le PM 198 a été administré par voie gastrique pendant 12 jours consécutifs à la dose de 200 mg/kg chez des souris obèses de 14 semaines.

Les résultats ont montré une chute de poids de 4,027 g, soit 10,75 %, et la consommation alimentaire des animaux traités a été fortement diminuée de 35,4 %.

Le Méclofénoxate, à la dose de 200 mg/kg, a été peu efficace (diminution de poids de 2,2 %).

Essai C

Effet anti-agrégant plaquettaire

Le PM 198, à la dose de 10 mg/kg, est actif comme antagoniste de l'agrégation plaquettaire observée sur les vaisseaux pie-mériens. [M.M.G. Bozeix, Int. Congress of Pharmacology, Paris, Juillet 1978].

Essai D

Activité diurétique

A la dose de 100 mg/kg P.O. le PM 198 a entraîné une excrétion urinaire de la surcharge hydrique de 37,7 % en 6 heures.

**Revendications** (pour les Etats contractants : BE, CH, DE, FR, GB, IT, LI, LU, NL, SE)

1. A titre de produits nouveaux, les bis-(phénoxyacétates substitués) de N-alkyl-dialcanolamines répondant à la formule générale

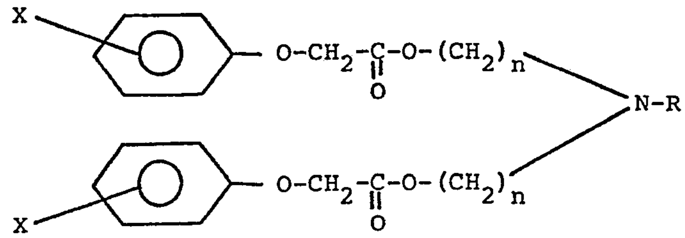

(I)

dans laquelle

— X est le chlore, le fluor ou le trifluorométhyle,

— n est compris entre 1 et 3 et est de préférence égal à 2,

— R est un groupe alkyle inférieur, tel que le groupe méthyle ou éthyle et les sels pharmaceutiquement acceptables desdits composés.

2. Composé selon la revendication 1, caractérisé en ce qu'il est le bis-(4-chloro-phénoxyacétate) de N-méthyl-diéthanolamine ou le bis-(3-trifluorométhyl phénoxyacétate) de N-méthyl-diéthanolamine.

3. Composé selon la revendication 2, caractérisé en ce qu'il se présente sous la forme de chlorhydrate, de méthane sulfonate, d'oxalate ou de maléate.

4. Composé selon la revendication 1, caractérisé en ce qu'il est le méthane sulfonate du bis-(4-chloro-phénoxyacétate) de N-méthyl-diéthanolamine.

5. Procédé pour l'obtention des composés selon l'une quelconque des revendications 1 à 4, caractérisé en ce qu'il consiste

1) à faire réagir l'acide phénoxyacétique de formule

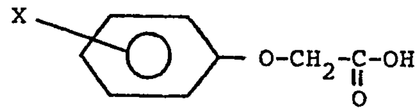

(II)

ou un de ses dérivés, avec une N-alkyl-dialcanolamine de formule

$$R—N—[(CH_2)_nOH]_2 \quad (III)$$

dans un rapport de l'acide à l'alcool sensiblement de 2 : 1.

2) à transformer éventuellement le composé obtenu en un sel pharmaceutiquement acceptable.

6. Procédé selon la revendication 5, caractérisé en ce que l'étape 1) est réalisée avec le chlorure de l'acide de formule II dans un solvant organique, tel que le benzène, à reflux du solvant.

7. Composition pharmaceutique, caractérisée en ce qu'elle contient, à titre d'ingrédient actif, un composé selon la revendication 1 en combinaison avec un véhicule pharmaceutiquement acceptable.

8. Composition pharmaceutique selon la revendication 7, caractérisée en ce qu'elle convient pour une administration orale ou parentérale.

9. Composition pharmaceutique selon l'une des revendications 7 ou 8, caractérisée en ce qu'elle contient 0,05 à 1,50 g de bis-(4-chloro-phénoxyacétate) de N-méthyldiéthanolamine selon la revendication 2.

**Revendications** (pour l'Etat contractant : AT)

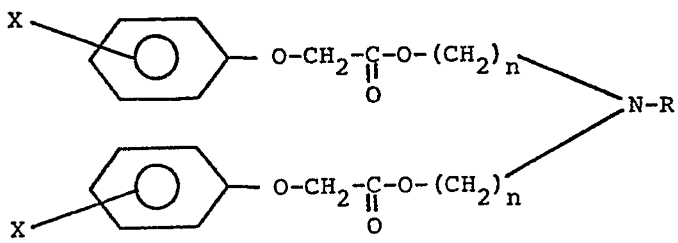

(I)

dans laquelle :

X est le chlore, le fluor ou le triluorométhyle,

n est compris entre 1 et 3 et de préférence égal à 2,

R est un groupe alkyle inférieur, tel que le groupe méthyle ou éthyle et des sels pharmaceutiquement acceptables desdits composés caractérisé en ce qu'il consiste :

caractérisé en ce qu'il consiste :

1) à faire réagir l'acide phénoxyacétique de formule

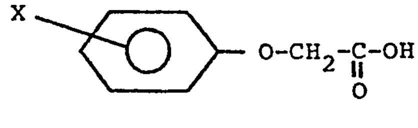

(II)

ou un de ses dérivés, avec une N-alkyl-dialcanolamine de formule

$$R—N[(CH_2)_nOH]_2 \quad (III)$$

dans un rapport de l'acide à l'alcool sensiblement de 2 : 1 et

2) à transformer éventuellement le composé obtenu en un sel pharmaceutiquement acceptable.

2. Procédé selon la revendication 1, caractérisé en ce que l'étape 1) est réalisée avec le chlorure de l'acide de formule II dans un solvant organique, tel que le benzène, à reflux du solvant.

3. Procédé selon la revendication 1 ou 2, caractérisé en ce qu'on prépare le bis(4-chlorophénoxyacétate) de N-méthyl-diéthanolamine ou le bis(3-trifluorométhylphénoxyacétate) de N-méthyl-diéthanolamine.

4. Procédé de préparation d'une composition pharmaceutique caractérisé en ce qu'on mélange un composé préparé selon la revendication 1, à titre d'ingrédient actif, avec un véhicule pharmaceutiquement acceptable.

5. Procédé selon la revendication 4, caractérisé en ce que le véhicule convient pour une administration orale ou parentérale.

6. Procédé selon l'une des revendications 3 ou 4, caractérisé en ce que l'ingrédient actif est constitué par 0,05 à 1,5 g de bis(4-chlorophénoxyacétate) de N-méthyl-diéthanolamine.

**Claims** (for the Contracting States : BE, CH, DE, FR, GB, IT, LI, LU, NL, SE)

1. As new products, the N-alkyldialkanolamine bis-(substituted phenoxyacetates) corresponding to the general formula

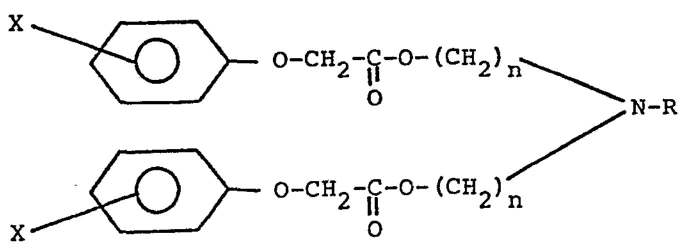

(I)

in which

— X is chlorine, fluorine or trifluoromethyl,

— n is between 1 and 3 and is preferably equal to 2, and

— R is a lower alkyl group such as the methyl or ethyl group, and the pharmaceutically acceptable salts of the said compounds.

2. Compound according to Claim 1, characterised in that it is N-methyldiethanolamine bis-(4-chlorophenoxyacetate) or N-methyldiethanolamine bis-(3-trifluoromethylphenoxyacetate).

3. Compound according to Claim 2, characterised in that it is in the form of the hydrochloride, methanesulphonate, oxalate or maleate.

4. Compound according to Claim 1, characterised in that it is N-methyldiethanolamine bis-(4-chlorophenoxyacetate)methanesulphonate.

5. Process for the preparation of the compounds according to any one of Claims 1 to 4, characterised in that it consists in

1) reacting the phenoxyacetic acid of the formula

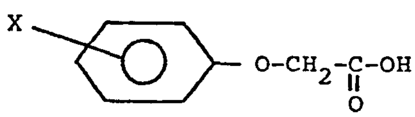

(II)

or one of its derivatives, with an N-alkyldialkanolamine of the formula

$$R—N—[(CH_2)_nOH]_2 \qquad (III)$$

in a ratio of the acid to the alcohol of approximately 2 : 1, and

2) if appropriate, converting the compound obtained to a pharmaceutically acceptable salt.

6. Process according to Claim 5, characterised in that step 1) is carried out with the chloride of the acid of the formula II, in an organic solvent such as benzene, under reflux of the solvent.

7. Pharmaceutical composition, characterised in that it contains a compound according to Claim 1 as the active ingredient, in combination with a pharmaceutically acceptable vehicle.

8. Pharmaceutical composition according to Claim 7, characterised in that it is suitable for oral or parenteral administration.

9. Pharmaceutical composition according to one of Claims 7 or 8, characterised in that it contains 0.05 to 1.50 g of N-methyldiethanolamine bis-(4-chlorophenoxyacetate) according to Claim 2.

**Claims** (for the Contracting State : AT)

1. Process for the preparation of N-alkyldialkanolamine bis(substituted phenoxyacetates) corresponding to the general formula :

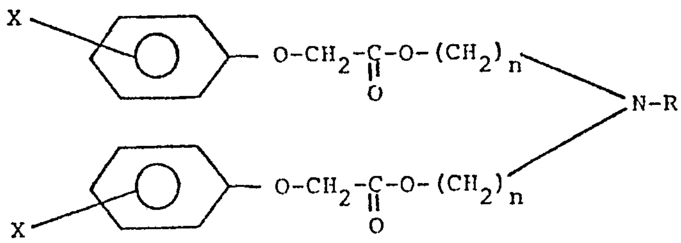

(I)

in which :

X is chlorine, fluorine or trifluorométhyl,

n is between 1 and 3 and is preferably equal to 2,

R is a lower alkyl group such as methyl or ethyl group, and the pharmaceutically acceptable salts of the said compounds characterized in that it consists in :

1) reacting the phenoxyacetic acid of the formula

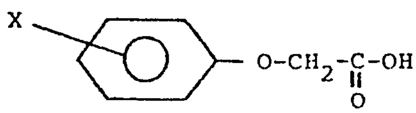

(II)

or one of its derivatives, with an N-alkyldialkanolamine of the formula :

$$R—N[(CH_2)_nOH]_2 \qquad (III)$$

in a ratio of the acid to the alcohol of approximately 2 : 1, and

2) if appropriate, converting the compound obtained to a pharmaceutically acceptable salt.

2. Process according to claim 1, characterized in that step (1) is carried out with the chloride of the acid of the formula II, in an organic solvent, such as benzene, under reflux of the solvent.

3. Process according to claim 1 or 2, characterized in that N-methyl diethanolamine bis(4-chlorophenoxyacetate) and bis (3-trifluoromethylphenoxyacetate) are prepared.

4. Process for the preparation of a pharmaceutical composition characterized in that a compound prepared as claimed in claim 1, as active ingredient, is combined with a pharmaceutically acceptable vehicle.

5. Process according to claim 4, characterized in that the composition is suitable for oral or parenteral administration.

6. Process according to one of claims 4 or 5 characterized in that active ingredient consists of 0,05 g to 1,5 g of N-methyldiethanolamine bis-(4-chlorophenoxyacetate).

**Ansprüche** (für die Vertragsstaaten : BE, CH, DE, FR, GB, IT, LI, LU, NL, SE)

1. Als neue Produkte die substituierten Bis-phenoxyacetate von N-Alkyldialkanolaminen entsprechend der allgemeinen Formel

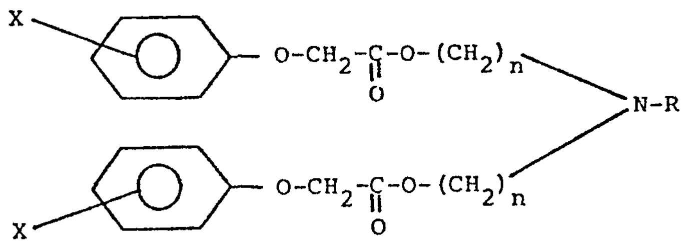

(I)

in der

— X chlor, Fluor oder Trifluoromethyl ist,

— n zwischen 1 und 3 liegt und vorzugsweise 2 ist,

— R ein niederer Alkylrest, z. B. ein Methyl- oder Ethylrest ist und die pharmazeutisch annehmbaren Salze der genannten Verbindungen.

2. Verbindung nach Anspruch 1, dadurch gekennzeichnet, daß sie das Bis-(4-chloro-phenoxyacetat) von N-Methyldiethanolamin oder das Bis-(3-trifluoromethylphenoxyacetat) von N-methyl-diethanolamin ist.

3. Verbindung nach Anspruch 2, dadurch gekennzeichnet, daß sie in Form des Hydrochlorids, des Methansulfonats, des Oxalats oder Maleats vorliegt.

4. Verbindung nach Anspruch 1, dadurch gekennzeichnet, daß sie das Methansulfonat des Bis-(4-chlorophenoxyacetats) von N-Methyldiethanolamin ist.

5. Verfahren zur Herstellung der Verbindungen nach irgendeinem der Ansprüche 1 bis 4, dadurch gekennzeichnet, daß es daraus besteht, daß man

1) Phenoxyessigsäure der Formel

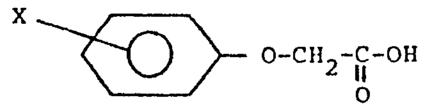

(II)

oder eines ihrer Derivate mit einem N-Alkyl-dialkanolamin der Formel

$$R—N—[(CH_2)_nOH]_2 \qquad (III)$$

in einem Verhältnis von Säure zu Alkohol von im wesentlichen 2 : 1 umsetzt,

2) die erhaltene Verbindung gegebenenfalls in ein pharmazeutisch annehmbares Salz umwandelt.

6. Verfahren nach Anspruch 5, dadurch gekennzeichnet, daß die Stufe 1) mit dem Chlorid der Säure der Formel II in einem organischen Lösungsmittel, z. B. Benzol, beim Rückfluß des Lösungsmittels durchgeführt wird.

7. Pharmazeutische Zubereitung, dadurch gekennzeichnet, daß sie als Wirkstoff eine Verbindung nach Anspruch 1 in Kombination mit einem pharmazeutisch annehmbaren Träger enthält.

8. Pharmazeutische Zubereitung nach Anspruch 7, dadurch gekennzeichnet, daß sie sich für die orale oder parenterale Verabreichung eignet.

9. Pharmazeutische Zubereitung nach einem der Ansprüche 7 oder 8, dadurch gekennzeichnet, daß sie 0,05 bis 1,50 g Bis-(4-chlorophenoxyacetat) von N-Methyldiethanolamin nach Anspruch 2 enthält.

**Ansprüche** (für den Vertragsstaat : AT)

1. Verfahren zur Herstellung von substituierten Bis phenoxyacetate von N-Alkyldialkanolaminen entsprechend der allgemeinen Formel

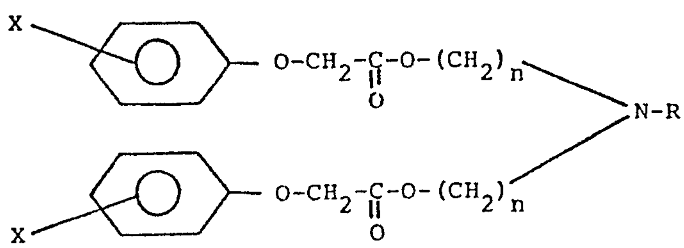

(I)

in der

X chlor, fluor oder trifluoromethyl ist,

n zwischen 1 und 3 liegt und vorzugsweise 2 ist,

R ein niederer Alkylrest, z. B. ein Methyl- oder Ethylrest ist und die pharmazeutisch annehmbaren Salze der genannten Verbindungen dadurch gekennzeichnet, dass es daraus besteht, dass man

1) Phenoxyessigsäure der Formel

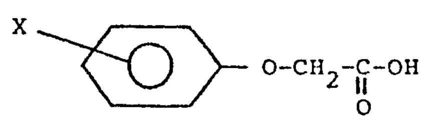

(II)

oder eines ihrer Derivate mit einem N-Alkyl-dialkanolamin der Formel

$$R—N[(CH_2)_nOH]_2 \qquad (III)$$

in einem Verhältnis von Säure zu Alkohol von im wesentlichen 2 : 1 umsetzt,

2) die erhaltene Verbindung gegebenenfalls in ein pharmazeutisch annehmbares Salz umwandelt.

2. Verfahren nach Anspruch 1, dadurch gekennzeichnet, dass die Stufe 1) mit dem Chlorid der Säure der Formel II in einem organischen Lösungsmittel, z. B. Benzol, beim Rückfluss des Lösungsmittels durchgeführt wird.

3. Verfahren nach Anspruch 1 oder 2, dadurch gekenzeichnet dass man Bis(4-chlorophenoxyacetat) von N-methyldiethanolamin oder das Bis(3-trifluoromethylphenoxyacetat)von N-methyldiethanolamin ist.

4. Verfahren zur Herstellung einer pharmazeutischen Zubereitung, dadurch gekennzeichnet, dass man eine nach Anspruch 1, 2 oder 3 hergestellte, Verbindung mit einem pharmazeutisch annehmbaren Träger mischt.

5. Verfahren nach Anspruch 4, dadurch gekennzeichnet, dass man ein für die orale oder parenterale Verabreichung geeignet Träger braucht.

6. Verfahren nach einem der Ansprüche 4 oder 5, dadurch gekennzeichnet, dass man als Wirkstoff 0,05 bis 1,50 g Bis(4-chlorophenoxyacetat) von N-Methyl-diethanolamin braucht.